# EUROPEAN PATENT APPLICATION

(11) **EP 2 141 247 A1**
(43) Date of publication of application: **06.01.2010**
(21) Application number: 08159457.4
(22) Date of filing: 01.07.2008
(51) Int. Cl.: C12Q 1/68

(54) **Markers for detection of Legionella strains**

(71) Applicant: Nederlandse Organisatie voor Toegepast- Natuurwetenschappelijk Onderzoek TNO, 2628 VK Delft (NL); Vitens N.V., 3528 AE Utrecht (NL)
(72) Inventor: Schuren, Frank Henri Johan, 3902 EB Veenendaal (NL); Atsma, Waatze Aize, 8925 AD Leeuwarden (NL)
(74) Representative: Hatzmann, Martin

(57) **Abstract**

The invention relates to a method of detecting *Legionella pneumophila* strains by hybridizing genomic DNA of a sample suspected to contain Legionella to N specific sequence markers, as identified by SEQ ID NO:N1 through SEQ ID NO:9 or homologues thereof.. The invention further relates to a kit of parts comprising an array and reference materials for performing a method of the invention.

## Description

### FIELD OF THE INVENTION

The invention relates to a method of identifying *Legionella pneumophila* strains, and in particular to a method of identifying a *Legionella pneumophila* strain as belonging to serogroup 1 or another serogroup. The invention further relates to markers for such an assay and a kit of parts comprising an array with said markers and reference materials for performing a method of the invention.

### BACKGROUND OF THE INVENTION

Legionnaires' disease is an acute pneumonic illness caused by Gramnegative bacilli of the genus *Legionella,* the most common of which is *Legionella pneumophila*.

Legionnaires' disease is initiated by inhalation, and probably microaspiration, of *Legionella* bacteria into the lungs. Although *Legionella* bacteria are ubiquitous in our environment, they rarely cause disease. A number of factors must occur simultaneously before legionnaires' disease is possible. These factors include the presence of virulent strains in an environmental site; a means for dissemination of the bacteria, such as by aerosolization; and proper environmental conditions allowing the survival and inhalation of an infectious dose of the bacteria by a susceptible host.

Water contaminated with a sufficient concentration of virulent *Legionella* bacteria can be aerosolized by water-cooled heat rejection devices such as air conditioning cooling towers, whirlpool spas, shower heads, water misters, and the like. Once the bacteria enter the lung, they are phagocytosed by alveolar macrophages and then grow intracellularly. The *Legionella* bacteria produce virulence factors that enhance phagocytosis. After sufficient intracellular growth, the bacteria kill the macrophage, escape into the extracellular environment and are then rephagocytosed by other macrophages. Within a few days after initial infection, the bacterial concentration in the lung increases considerably. The resulting infiltration of the alveoli by neutrophils, additional macrophages and erythrocytes results in capillary leakage and edema and the chemokines and cytokines released by the macrophages help trigger a severe inflammatory response, which may be fatal.

More than 49 different *Legionella* species, encompassing 70 serogroups, have been described since its first discovery in 1977, 20 of which have been reported to infect humans. *L. pneumophila* contains at least 16 different serogroups. *L. pneumophila* serogroup 1 caused the 1976 Philadelphia outbreak and is the cause of 70% to 90% of all cases of legionnaires' disease. In the major outbreaks such as those originating in Bovenkarspel, The Netherlands in 1999, in Barrow-in-Furness in Cumbria, England in 2002 and near Harnes in Pas-de-Calais, France in 2003/2004, serogroup 1 strains could be detected in the majority of the patients.

*L. pneumophila* serogroup 1 can be further divided into multiple subtypes using a variety of serologic, other phenotypic and genetic methods. One particular subtype of *L. pneumophila* serogroup 1 causes the majority of cases of legionnaires' disease due to *L. pneumophila,* and 85% of the cases due to *L. pneumophila* serogroup 1; this subtype is distinguished by its reactivity with a particular monoclonal antibody, and it is variously termed Pontiac, the Joly monoclonal type 2 (MAb2), or the Dresden monoclonal type 3/1 (MAb 3/1) monoclonal subtype.

Most clinical microbiology laboratories are capable of identifying *Legionella* bacteria to the genus level by detection of their typical colony morphology, Gram stain appearance, and various other standard microbiology identification techniques. Identification of *L. pneumophila* serogroup 1, the most common clinical isolate can be accomplished by sophisticated clinical microbiology laboratories using relative simple serologic testing. However, it should be stressed that serogroup 1 strains are not the only virulent serotypes

Identification of other *L. pneumophila* serogroups, and other *Legionella* species, is often much more difficult. This is because these bacteria are relatively inert in the use of commonly tested biochemical substrates, and they require sophisticated phenotypic, serologic and molecular testing. Reference laboratory-based phenotypic testing of bacteria, including determination of fatty-acids and ubiquinones and protein electrophoresis, can often be used to identify the bacteria. Definitive identification is based on both immunologic detection of surface antigens and bacterial DNA sequencing. Also, DNA typing by pulsed field electrophoresis of DNA restriction fragments is useful for identification purposes.

Diagnosis of the infecting agent from sample material (such as water that is suspected of contamination, or of clinical samples) on selective media is currently the most reliable means of diagnosis. However, cultivation is slow. Direct fluorescent antibody (DFA) stains for the visualization of *Legionella* species in clinical specimens are commercially available for a limited number of species. Assays for the detection of *L. pneumophila* serogroup 1 antigen in urine have a sensitivity of 70% and a specificity of nearly 100%. Also, an immunochromatographic assay for the rapid qualitative detection of *L*. *pneumophila* serogroup 1 antigen (Legionella NOW; Binax, Portland, Maine) in urine specimens has become available that uses rabbit anti-*L*. *pneumophila* serogroup 1 antibody as the capture component and rabbit anti-*L*. *pneumophila* serogroup 1 antibody conjugated to colloidal gold as the detection component. The assay provides a test result in 15 min and is intended to aid in the presumptive diagnosis of Legionnaires' disease caused by *L. pneumophila* serogroup 1 in conjunction with culture and other methods. Preliminary performance data for the immunochromatographic assay report a sensitivity of 95% and a specificity of 95%. Thus, so far, commercially available tests for *Legionella* urinary antigen detect only *L. pneumophila* serogroup 1, while the specificity of the assays cannot prevent the occurrence of false positive and false negative reactions.

DNA probe techniques, which produce fewer false positive reactions then immunological detection methods, may be used to detect the presence of one or more multiple *Legionella* species. However, a drawback of such DNA methods is that they cannot differentiate between virulent and non-virulent strains, presumably because the virulence trait is multi-genic. Further, they are not able to adequately identify *Legionella* strains if more than one strain is present in the sample.

Following severe outbreaks, many national authorities have implemented legislation and water quality standards for water supplies and/or codes of practice for management and operation of cooling towers and warm water storage facilities. Such standards and codes require frequent monitoring of drinking water distribution systems and swimming-pool water facilities, and upon exceeding a certain number of *Legionella* bacteria per liter, rigorous measures are taken, such as closure and evacuation of hotels, sports facilities or nursing homes. Most experts however, consider that a large number of *Legionella* bacteria detected are harmless and non-virulent. However, there is at present no assay system available, except for the tedious assay involving culturing of individual bacteria, which takes about 7 days, to correctly identify possible harmful strains if more than one bacterial strain is present in the sample. The availability of a test that is capable of reliably detecting DNA markers from a specific single *Legionella* strain would be highly favorable.

It is an object of the present invention to provide for a method capable of identifying multiple strains of *L. pneumophila,* and especially to discriminate between serogroup 1 strains, strains within this serogroup 1 and strains from other serogroups.

### SUMMARY OF THE INVENTION

The present inventors have now found a method for identifying serogroup 1 *Legionella* strains from non-serogroup 1 strains. The method involves an hybridization assay with specific genetic markers. The inventors further have been able to discriminate between subgroups in the serogroup 1 strains.

In one embodiment of the present invention, the group of molecular markers comprises the marker 11B3, indicated in SEQ ID NO:1 or sequences which are highly homologous therewith.

In a further embodiment of the method of the present invention, the group of molecular markers comprises the 8 markers (5G5, 9G2, 8E11, 30A9, 9F12, 35C6, , 7C10 and 12A12) indicated in SEQ ID NO:N2 to SEQ ID NO: 9, or homologues thereof.

In a further embodiment of the method of the present invention, the sample nucleic acid is derived from a mixed culture of *Legionella pneumophila.* Alternatively, the sample can be derived from any medium which is suspected of *Legionella* infestation, such as aqueous samples from water pipes, surface water, drink water, showers, baths, fountains, etc.

In a specific embodiment, a method of the invention is used for determining the presence of a plurality of Legionella strains, the method involving a hybridization assay with at least two specific genetic markers selected from the group of markers as depicted in SEQ ID NO:1 - SEQ ID NO:9 or homologues thereof, wherein said markers are specific for a different strain.

A kit of parts, said kit comprising an array as defined herein together with reference nucleic acids as defined herein. Preferably, said kit also comprises software to calculate the result of the test.

### DETAILED DESCRIPTION OF THE INVENTION

The term "*Legionella pneumophila* strain" as used herein refers to the descendants of a single isolate of *Legionella pneumophila* cells in pure culture and to a taxonomic level of said pure culture below the level of the species *Legionella pneumophila.*

The term "pathogenic" in relation to a *Legionella* strain refers to a microorganism capable of causing disease (including infection) or morbid symptoms in humans or other animal hosts.

The term "array" refers to an array of individual fragments of DNA or oligonucleotides that are bound to a substrate such as a microscope slide. The purpose of an array experiment is to determine the amount of matching nucleic acid fragments in a particular sample. The target nucleic acid fragments are extracted from the cells or tissues of interest, optionally converted to DNA, and labeled. They are then hybridized to all the DNA or oligonucleotide spots on the array. Matches (i.e. the spots where hybridization has taken place) are identified using antibody detection, optionally combined with precipitation, chromatography, colorimetry, and/or phosphorescent or fluorescent imaging. The data resulting from an array experiment are a list of measurements of spot intensities. A preferred array of the invention is an array which has less than 1000, preferably less than 100, more preferably less than 10 spots, and which contains at least the DNA sequences of SEQ ID NO:1 and SEQ ID NO:2 Another preferred array of the invention is an array which has less than 1000, preferably less than 100, more preferably less than 10 spots, and which contains at least five sequences selected from the sequences as presented in SEQ ID NO:1 through SEQ ID NO:9, with the proviso that the number of spots is at least equal to the number of sequences that the array comprises.

The term "nucleic acid" as used herein, is interchangeable with the term "polynucleotide", and refers to a nucleotide multimer or polymeric form of nucleotides having any number of nucleotides, e.g., deoxyribonucleotides or ribonucleotides, or compounds produced synthetically (e.g. PNA as described in U.S. Pat. No. 5,948,902 and the references cited therein) and can be either double- or single-stranded. A polynucleotide can hybridize with other polynucleotides in a sequence specific manner, e.g. can participate in Watson-Crick base pairing interactions. The term also includes modified, for example by methylation and/or by capping, and unmodified forms of the polynucleotide

The terms "deoxyribonucleic acid" and "DNA" as used herein mean a polymer composed of deoxyribonucleotides.

The term "oligonucleotide" refers to a short sequence of nucleotide monomers (usually 6 to 100 nucleotides) joined by phosphorous linkages (e.g., phosphodiester, alkyl and aryl-phosphate, phosphorothioate), or non-phosphorous linkages (e.g., peptide, sulfamate and others). An oligonucleotide may contain modified nucleotides having modified bases (e.g., 5-methyl cytosine) and modified sugar groups (e.g., 2'-O-methyl ribosyl, 2'-O-methoxyethyl ribosyl, 2'-fluoro ribosyl, 2'-amino ribosyl, and the like). Oligonucleotides may be naturally-occurring or synthetic molecules of double-and single-stranded DNA and double- and single-stranded RNA with circular, branched or linear shapes and optionally including domains capable of forming secondary structures (e.g., stem-loop, pseudo knots and kissing loop structures).

The term "nucleotide sequence homology" as used herein denotes the presence of homology between two polynucleotides. Polynucleotides have "homologous" sequences if the sequence of nucleotides in the two sequences is the same when aligned for maximum correspondence. Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence homology" for polynucleotides, such as 50, 60, 70, 80, 90, 95, 98, 99 or 100 percent sequence homology may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by visual inspection. Readily available sequence comparison and multiple sequence alignment algorithms are, respectively, the Basic Local Alignment Search Tool (BLAST) (Altschul et al., 1990; Altschul et al., 1997) and ClustalW programs, both available on the internet. Other suitable programs include, but are not limited to, GAP, BestFit, PlotSimilarity, and FASTA in the Wisconsin Genetics Software Package (Genetics Computer Group (GCG), Madison, WI, USA) (Devereux et al., 1984).

As used herein, "substantially complementary" means that two nucleic acid sequences have at least about 65%, preferably about 70%, more preferably about 80%, even more preferably 90%, and most preferably about 98%, sequence complementarity to each other. This means that primers and probes must exhibit sufficient complementarity to their template and target nucleic acid, respectively, to hybridise under stringent conditions. Therefore, the primer and probe sequences need not reflect the exact complementary sequence of the binding region on the template and degenerate primers can be used. For example, a non-complementary nucleotide fragment may be attached to the 5'-end of the primer, with the remainder of the primer sequence being complementary to the strand. Alternatively, non-complementary bases or longer sequences can be interspersed into the primer, provided that the primer has sufficient complementarity with the sequence of one of the strands to be amplified to hybridize therewith, and to thereby form a duplex structure which can be extended by the polymerising means. The non-complementary nucleotide sequences of the primers may include restriction enzyme sites. Appending a restriction enzyme site to the end(s) of the target sequence would be particularly helpful for cloning of the target sequence. A substantially complementary primer sequence is one that has sufficient sequence complementarity to the amplification template to result in primer binding and second-strand synthesis. The skilled person is familiar with the requirements of primers to have sufficient sequence complementarity to the amplification template.

The term "hybrid" in the context of nucleic acids refers to a doublestranded nucleic acid molecule, or duplex, formed by hydrogen bonding between complementary nucleotide bases. The terms "hybridise" or "anneal" refer to the process by which single strands of nucleic acid sequences form double-helical segments through hydrogen bonding between complementary bases.

The term "probe" refers to a single-stranded oligonucleotide sequence that will recognize and form a hydrogen-bonded duplex with a complementary sequence in a target nucleic acid sequence analyte or its cDNA derivative.

The term "primer" as used herein refers to an oligonucleotide which is capable of annealing to the amplification target allowing a DNA polymerase to attach, thereby serving as a point of initiation of DNA synthesis when placed under conditions in which synthesis of primer extension product is induced, i.e., in the presence of nucleotides and an agent for polymerisation such as DNA polymerase and at a suitable temperature and pH. The (amplification) primer is preferably single stranded for maximum efficiency in amplification. Preferably, the primer is an oligodeoxyribonucleotide. The primer must be sufficiently long to prime the synthesis of extension products in the presence of the agent for polymerisation. The exact lengths of the primers will depend on many factors, including temperature and composition (A/T en G/C content) of primer. A pair of bi-directional primers consists of one forward and one reverse primer as commonly used in the art of DNA amplification such as in PCR amplification.

It will be understood that "primer", as used herein, may refer to more than one primer, particularly in the case where there is some ambiguity in the information regarding the terminal sequence(s) of the target region to be amplified. Hence, a "primer" includes a collection of primer oligonucleotides containing sequences representing the possible variations in the sequence or includes nucleotides which allow a typical base pairing.

The oligonucleotide primers may be prepared by any suitable method. Methods for preparing oligonucleotides of specific sequence are known in the art, and include, for example, cloning and restriction of appropriate sequences, and direct chemical synthesis. Chemical synthesis methods may include, for example, the phospho di- or tri-ester method, the diethylphosphoramidate method and the solid support method disclosed in e.g. U.S. Pat. No. 4,458,066. The primers may be labelled, if desired, by incorporating means detectable by for instance spectroscopic, fluorescence, photochemical, biochemical, immunochemical, or chemical means.

Template-dependent extension of the oligonucleotide primer(s) is catalysed by a polymerising agent in the presence of adequate amounts of the four deoxyribonucleotide triphosphates (dATP, dGTP, dCTP and dTTP, i.e. dNTPs) or analogues, in a reaction medium which is comprised of the appropriate salts, metal cations, and pH buffering system. Suitable polymerizing agents are enzymes known to catalyse primer- and template-dependent DNA synthesis. Known DNA polymerases include, for example, E. coli DNA polymerase I or its Klenow fragment, T4 DNA polymerase, and Taq DNA polymerase. The reaction conditions for catalysing DNA synthesis with these DNA polymerases are known in the art.

The products of the synthesis are duplex molecules consisting of the template strands and the primer extension strands, which include the target sequence. These products, in turn, serve as template for another round of replication. In the second round of replication, the primer extension strand of the first cycle is annealed with its complementary primer; synthesis yields a "short" product which is bound on both the 5'- and the 3'-ends by primer sequences or their complements. Repeated cycles of denaturation, primer annealing, and extension result in the exponential accumulation of the target region defined by the primers. Sufficient cycles are run to achieve the desired amount of polynucleotide containing the target region of nucleic acid. The desired amount may vary, and is determined by the function which the product polynucleotide is to serve.

The PCR method is well described in handbooks and known to the skilled person.

After amplification by PCR, the target polynucleotides may be detected by hybridisation with a probe polynucleotide which forms a stable hybrid with that of the target sequence under stringent to moderately stringent hybridisation and wash conditions. If it is expected that the probes will be essentially completely complementary (i.e., about 99% or greater) to the target sequence, stringent conditions will be used. If some mismatching is expected, for example if variant strains are expected with the result that the probe will not be completely complementary, the stringency of hybridisation may be lessened. However, conditions are chosen which rule out non-specific/adventitious binding. Conditions which affect hybridisation, and which select against non-specific binding are known in the art, and are described in, for example, Sambrook et al., (2001). Generally, lower salt concentration and higher temperature increase the stringency of binding. For example, it is usually considered that stringent conditions are incubations in solutions which contain approximately 0.1xSSC, 0.1% SDS, at about 65°C incubation/wash temperature, and moderately stringent conditions are incubations in solutions which contain approximately 1-2xSSC, 0.1% SDS and about 50°-65°C incubation/wash temperature. Low stringency conditions are 2xSSC and about 30°-50°C.

The terms "stringency" or "stringent hybridisation conditions" refer to hybridisation conditions that affect the stability of hybrids, e.g., temperature, salt concentration, pH, formamide concentration and the like. These conditions are empirically optimised to maximize specific binding and minimize non-specific binding of primer or probe to its target nucleic acid sequence. The terms as used include reference to conditions under which a probe or primer will hybridise to its target sequence, to a detectably greater degree than other sequences (e.g. at least 2-fold over background). Stringent conditions are sequence dependent and will be different in different circumstances. Longer sequences hybridise specifically at higher temperatures. Generally, stringent conditions are selected to be about 5°C lower than the thermal melting point (Tm) for the specific sequence at a defined ionic strength and pH. The Tm is the temperature (under defined ionic strength and pH) at which 50% of a complementary target sequence hybridises to a perfectly matched probe or primer. Typically, stringent conditions will be those in which the salt concentration is less than about 1.0 M Na+ ion, typically about 0.01 to 1.0 M Na+ ion concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions or "conditions of reduced stringency" include hybridisation with a buffer solution of 30% formamide, 1 M NaCl, 1% SDS at 37°C and a wash in 2x SSC at 40°C. Exemplary high stringency conditions include hybridisation in 50% formamide, 1 M NaCl, 1% SDS at 37°C, and a wash in 0.1x SSC at 60°C. Hybridisation procedures are well known in the art and are described by e.g. Ausubel et al., 1998 and Sambrook et al., 2001.

The term "fragmented genomic DNA" refers to pieces of DNA of the genome of a cell that are the result of the partial physical, chemical or biological break-up of the lengthy DNA into discrete fragments of shorter length.

The term "hybridization pattern" refers to the list of measurements of spot intensities obtained after hybridizing the array with a target nucleic acid.

The term "nucleotide" is used to denote a deoxyribonucleoside, a ribonucleoside, or a 2'-O-substituted ribonucleoside residue

The term "molecular marker" generally refers to markers identifying variation at the level of DNA and is herein used to refer to a mutation (of any type) or nucleotide sequence which has a scorable or selectable relation with the phenotype of serogroup 1 or any other serogroups, or with any special subgroup within a serogroup. (and hence can "mark" a region of the chromosome).

An 'incompatible marker pair' is a pair of markers that is specific for one serogroup or subgroup within a serogroup of *Legionella,* while the other member of the pair of markers is specific for the rest of the serogroup(s) or subserogroup(s).

Now, a marker, designated 11B3 (SEQ ID NO: 1) has been found, that is specific for *Legionella* subgroups 2-14 and which does not occur in subgroup 1. In this way, it can be used as a marker discriminating bacteria from subgroup 1 from bacteria from the other subgroups. Conversely, a marker designated 19H4 (SEQ ID NO: 10) has been found which is specific for serogroup 1. Thus, if a mixture of *Legionella* strains is present in a sample, it can easily be detected whether such a sample harbours members of the serogroup 1 phenotype and/or of other serogroup phenotypes.

Next, it has also been shown to be possible to find incompatible marker pairs within serogroup 1. A first example is the pair of markers formed by marker 5G5 (SEQ ID NO: 2), which is unique for serogroup 1A and any of the markers 9G2 (SEQ ID NO: 3), 8E11 (SEQ ID NO: 4) and 30A9 (SEQ ID NO: 5) that are unique for serogroup 1B. Of the three markers for serogroup 1B, the marker 9G2 is preferred.

Further incompatible marker pairs are the pairs 9F12 - 35C6, 24D9 - 25B6 and 7C10- 12A12.

A detection whether a test sample contains multiple species of *Legionella* would be feasible with the above described markers. Such a detection would be fast and reliable, since only minor amounts of sample material are needed to perform the hybridization tests and thus no culturing of the bacterial strains has to be performed. Further, hybridization tests are reliable and, as is shown in the examples, the tests with the above described markers give robust results.

In another embodiment of the present invention, the array comprises at least three, at least five, at least seven of the nine sequences as depicted in SEQ ID NO:1 through SEQ ID NO:9 or all of these nine sequences. The sequences of markers given in the sequence listing are about 1k nucleotides long. However, this does not necessarily mean that the complete sequence as shown in any of the SEQ ID's needs to be present on the array: the assay can be been performed with sequences, which are parts thereof. It should, however, be understood, that a minimal length is necessary for obtaining a correct and distinctive hybridization with any sample nucleic acid. Thus, the length of these part(s) of the sequences should range from about 50 to about 2000 nucleotides, i.e. the sequences can be shorter or longer than those presented in the sequence listing, but for a sufficient hybridization to occur they should be at least 50 nucleotides long. Longer sequences than those depicted can be obtained by hybridizing the genome from a *Legionella* species and, if hybridization occurs with the presented sequence, excise a larger part of the sequence from the genomic DNA.

Further, hybridization allows for some mismatches between the sequence on the array and the nucleotide sequence of the sample. Therefore, any nucleotide sequence or part thereof (as defined above) which has a homology of at least 70%, preferably at least 80%, more preferably at least 90%, more preferably at least 95%, even more preferably at least 99% with the sequences of SEQ ID NO: 1 through SEQ ID NO: 9 is applicable in and intended within the scope of the present invention.

In the current invention the term "homologues" is used to indicate both parts of the sequences of the invention of at least 50, but preferably at least 100, more preferably at least 200, more preferably at least 500 and most preferably at least 1000 nucleotides, and sequences with have a degree of homology of at least 70%, preferably at least 80%, more preferably at least 90%, more preferably at least 95%, even more preferably at least 99% with the sequences of SEQ ID NO:1 through SEQ ID NO:9.

The above discussed markers, or fragments thereof may be spotted on a surface to provide for a DNA micro-array. In order to facilitate coupling of the fragments, the surface of the array (e.g. the slide, the surface of which may i.a. be glass, gold, etc.) may be modified. Spotting may occur by any method available, for instance by using ElectroSpray Ionization (ESI) micro-array printing. After spotting of the markers or fragments thereof, the slide surfaces may be blocked to prevent further attachment of nucleic acids, e.g. by treatment with boro-anhydride in case of formaldehyde modified glass-slide surfaces.

To facilitate detection of successful hybridization, the gDNA is suitably labeled, preferably with a compound which is uniquely detectable by an antibody. Such a compound can for instance be biotin. Labeling of nucleotides with biotin and subsequent detection through antibodies specific for biotin, has been sufficiently described in the literature and will be routine experimentation for a person skilled in the art. Alternatively, the nucleotides are fluorescently labeled (e.g. by using CyTM labels [Amersham Pharmacia Biotech]). Fluorescent labeling kits are commercially available from various manufacturers. In order to be able to judge the signals caused by the hybridization of the marker sequences with sample nucleic acid, preferably the array also comprises reference nucleotide sequences, which can serve as positive and negative controls. As negative controls, sequences should be used of about the same length as the average length of the markers, but which will not hybridize with any nucleotide sequence in the sample, i.e. sequences, which do not occur in *Legionella.* As positive control, a sequence should be used which will be present in (nearly) all samples to be tested. For this purpose preferably the *Legionella* 30S ribosomal protein S21 (Cazalet,C., Rusniok,C., Bruggemann,H., Zidane,N., Magnier,A.,Ma,L., Tichit,M., Jarraud,S., Bouchier,C., Vandenesch,F., Kunst,F., Etienne,J., Glaser,P. and Buchrieser,C. Nat. Genet. 36 (11), 1165-1173 (2004)) or a conserved sequence of one of the housekeeping enzymes should be used.

The average size of sample nucleic acid has an effect on the signal distribution on the array. Larger sample molecules comprise more information and are thus more likely to find a suitable hybridization partner in more of the spots. Reducing the average size of the sample nucleic acid can reduce this phenomenon. On the other hand, when the sample nucleic acid is too small, the nucleic acid fragments in the sample contain too little genetic information and also find suitable hybridization partners in many spots. The average size of the fragments in the sample nucleic acid is preferably between about 30 and 3000 nucleotides. More preferably, the average size of the fragments in the sample nucleic acid comprises a size of between about 50 and 1000 nucleotides, more preferably between about 100 and 500 nucleotides.

In a method for detecting a *L. pneumophila* strain, the sample nucleic acid may represent the whole or a part of the sample genome. Preferably, at least those parts of the genome are present in which the presence of molecular markers as defined herein is to be detected. This can be achieved by randomly digestion of the genomic DNA of the sample to fragments of about 1.5 kb or by physically fragmenting the DNA (e.g. by shearing) to form fragments of about that size. In a preferred embodiment a PCR amplification step is performed on either the intact genomic DNA or on the fragmented DNA with primers that specifically cause amplification of one or more of the sequences of the invention. Alternatively, the DNA can be randomly (primer) labeled using Klenow DNA polymerase (BioPrime kit Invitrogen) according to the manufacturer's instructions.

The fragmented sample DNA is then brought into contact with the array of the invention, which contains at least the two marker sequences of SEQ ID NO:1 and SEQ ID NO:10 or fragments thereof, or homologues of said markers or fragments thereof. In addition or alternatively the array comprises at least one of the following combinations of two marker sequences: SEQ ID NO:2 and SEQ ID NO:3; SEQ ID NO:2 and SEQ ID NO:4; SEQ ID NO:2 and SEQ ID NO:5; SEQ ID NO:6 and SEQ ID NO:7; SEQ ID NO:8 and SEQ ID NO:9, or fragments thereof, or homologues of said markers or fragments thereof. Hybridization of the sample nucleotides with the marker sequences and the hybridization signals with optional positive and negative control sequences is then determined.

### EXAMPLES

### DNA isolation of sample

In the present Example a total of 144 samples from different strains were selected from the collection of strains of the Streeklaboratorium Kennemerland in Haarlem, The Netherlands. Of these 144 samples, a total of 74 samples were isolated from hospital patients (pathogenic or clinical strains) and a total of 70 samples was isolated from industrial and public water supply systems, and were not detected in humans (environmental strains).

### Labeling and hybridisation of genomic DNA

All strains were cultivated after which genomic DNA was isolated from these strains. The DNA was then randomly (primer) labeled using Klenow DNA polymerase (BioPrime kit Invitrogen) according to the manufacturer's instructions.

Cy3 labeled marker sequences were prepared. The DNA sample consisted of the DNA of the test strain and was labeled with Cy5. Both labeled samples were hybridized simultaneously to a microarray. Upon scanning and image analysis, the ratio of the Cy5/Cy3 emission values was calculated for each spot on the microarray. These ratio's served as data input for further data analysis.

Sequence ID's:

SEQ ID NO 1: 11B3

GAATTCGTCGGAAACCAATCGGCGGTTTTTTTAAGCCCTCACATGAATCA
ACGGGTGGAGTCCAGCCTAAGTCTGAAATAATCTTTGAACTATCAATTTCC
AGAGAACTCAGCAAGCGAGTATTTAAACTGCCCATTCCTGTGATCTTGAA
CAGGCCGGCTAAAAAAGTAGTAGGGAAGTGATACAATCTTAAATTGATTT
TCATATTTTGGGCTAGTAAGGATAACAAATCCGCTAAAGACCAAGATTCAT
TATCTGCTACAAGATAAAGTTGATTTGCTGCTTTAGGATGTGTCATTACCA
TTAGAATAGCTGAAACCAAGTTATCAATAAAAATAAAACTTCTCTTATTCT
TTATACTGGCAAAAGGCAATGGCCATCCTTTTTGTACCATACCCAATAATT
TTAAAAAATTGGCTTTTACTCCAGGTCCATAAACAAGTGGAGGCCTCAAG
ATAACCACTTCCATGTCACTATTTTCACTAATCTCACGCAAATAGAGTTCC
GCTTCTAACTTACTCTGAGCATAAGAATCTTTTGGTTGGGCTGAATTTTTC
TCAGAAAATGGGCTCTGAGGTAAGCTTACTTCTCCATTTACCTTAATAGTA
CTTAAAAATACAAATCGCTTTACTTTGTGTTTTGCAGCTTGTTGAGCAAAA
TTCTTGGTGGCGATGCTGTTTATTTTGCAATACTCTTCCAGAGAGGAGGA
GGTATCCTCTCTCATAACATGGACTCGCGCAGCCAGATGTATGACAATAT
CGATTCCATTCAAAGCTTCTGACCCATCCGTTACTTGCTCCAGCTTATCAA
TTTCAACTTGCTCAACAGACATGTATTGATTTTTTTGCCACACAGCACAAC
GAACCTCATACCCTGCAGCAATAAGAACCGGGAACCAAGCTACTGCCCAT
AAAACCTGGTGCGCCCGTCACTAATACTTTTGTCACTATATTACCTATGGC
TT

SEQ ID NO 2: 5G5

TGACGAATCAGAGATTCGTCTTATTAACCTTTTTCTATGGAAAAACGCATG
gTGGGACAAAGTATTCTGCTGAACATCACCTAAATGAATATAaaAAGAATC
TGGAAGATGATATAAAGGCAATCGGTGTTCAAAGAAAAATTTCACCAAAT

GCCTATGAAGATCTATTAAAAGAGAAAAAAGAGCGCCTGGAACAAATCGA
AAAATATATTGATTTAATCAAAGTACTCAAAGAGAAATTTGATGAGCAAAG
AGAAATAGACAAACTACGCACAGAAGGAATCCCGCAATTACCTTCTGGAG
TAAAGGAAGTCATTAAATCTTCTGAAAATGCCTTTGCTTTAAGGCTTTCCC
CGGATAGACCAGACTCATTTACCCGCTTTGATGATCCTCTGTTTAGTCTCA
AAAGAAACAGATCTCAATATGAAGCTGGGGGATACCAACGGGCAACTGAT
GGATTAGGGGCTCGTTTGCGTTCTGAGCTTCTACCACCAGATAAAGACAC
TCCTATTGTTTTTAATAAAAAATCACTGAAGGACAAAATCGTAGATTCTGT
TTTAGTGCAACTTGACAAAGATTTAATACCAAAGATGGCGATCGTGGTCA
AAAATTTGAGGATATAAAAAAACTCGTTCTAGAAGAGTACAAGAAAATTG
ATTCCGACCTTCAGGTGGATGAGGATACCTATCACCAACCCCTTAACTTG
GATTATTTGGAAAACATAGCATGTACGTTAGATGAACACTCCCCTCGCAAA
GAATGAGTTTATGGAATTATGGGTGCCCCCAACCGAAGCTGATTATGGGC
CCAAAAAAGGAAAGGGCAACGTGGAACTGAACAACTTAGTGGTATTTATT
AACAACCCCAAGGAATATAAAATTTGAATCTGAATACAAATACCATGGCCA
CTTACGGTCCAATATTCAATAACTGAAGATTATTTTTATTGGCAAACCCC
ACCACCTTCTCAAAGTCCAAATTTCGGTGAATTTTTTGCAATAAACACCCC
CCAGGC

SEQ ID NO 3: 9G2

CGCGAACCGAGAACCGTTCGGAGGCGATGAGAACGGAACCGATAACACT
CGTCCAAACAGCAGTTGAATTAAGATCATTCCCCTACGGAAATACTCCACC
ACGGCGAAGCTAGGCATTCCCGCTGAGCAACAGCTCGACTCGATTATAAA
AAACAACTCGGAAGAAGACATAAAAGCCATCAATAGCCAAAAAGGATAT
CACGTTCATCGCTTATACCGATTCTCGTTAAGGGAAAAAAAGGAACGACT
TAAACAAATTCGAAAAATACATTCGAGTTAATTCAACGTACTCAAAACAGA
ATATGATCACAACGGTGAAATAATGACTATAAAAAATAACATTATTCCACC
TCTACCTTTCCGGATTAAACCAAATAATCCAGTCTTCTGAGAATGCTGGTG
CTGTAAGACTGTCCCCAGATCGCCCTGATTTGGCAACCAGTTTTAAAAACC

CTTTATTCAGGTTAAATAGACATTATGAAACCAGCGATTATAAACTGACAG
AAGGCTTAGGGGTTCGCCTCAGTTCAACGCTCTTGCCAGATCCTGAAACT
CCTACGCCAATTAATAGAAAATCACAAAAAGAAAAAATTGTAGAAACTGTT
TTAGCTAAATTTCAACCAGAAAAAATAGCTGAACCAGACCGTGACCAAAA
ATTGAAAGAGCTAAAAGCCCTTTCTTCAGAAGAGCTTGTAAAAATTGACTC
CAATTTGTCAGTGGATATAAGCCATGATAAACAAGAGACAAACTACGACT
ATCTGGAAAATATGATGGNCATGGATGAAGATAGTTTCATCAGGAATGGG
GTTTGATCTATTCTCACAGCGACAGTAGATTCGAGCGCTTGGGGTTACTC
AATCCCGCAGTCCTTTCTATGATGGCGCCAAAGAATAAACAAAAAGACGA
TGCGGATAAAATGTCCATCAAAGTACAATACCTAATAGCCGAGGCTAATTT
TTATTGTAAAACGAACAAATATTCCATGCTAACTTTCGGGAAATTTTTTG
ACAAGGAGCCTCATGCCACTGAAATTGCGAAAAGAGTCAAAGAAGGATTG
GTGCAAGGAGTAGATATAGAGCCTATCATATACAATTACATCAACAGCAA
CCATGCAGAGCTTGGATTAAAATCCCCTTTGACTGCCAAACAACAACAAG
AAATCACCGATAAATTTTCGCAACATTACAATACCATTAAGGACTCCCCTC
ATTTTGATGAATTTTTATCGCTGATCCTGATAAGAAGGAAATATATTTA
CCCATCAGGGCAGACTCAGTTGCCATTTCCTTGACTTCTTTGCCCGACAAA
CTAACGCCAAACATCTTTTGGGAGAACTTGACGGTCACGCAGAAGCATTA
CTGGAAGGAACTTCAAATCGCTTAAATCATAAAAATGAAATTGTGGCGGA
AGGATATGAGAAAATAGAGCAATTTAACAAGAAGTAGTAAGGCTTCTGGC
TGAAAATAACCCAAAGAATGAACGATTCAGCTG

SEQ ID NO 4: 8 E11

GAATCGTCTCGATCTGGTAGCCTCAAGGATTCTGACATTGCCTCCTTCGCC
AGCGCGTCGCACGAATACAAGGGCTGCTCGTCATTAACCCCAAACGGTGC
CGTCGTGATCGTTATCACCCGGTCGCCATTCCTTACACCAACGACGGAGG
ATCGTGCTTTTCCAGTCGTTTTGCGTGAGCCAGTCTTTGCGCAAAAAAACC
AGATTTTCCAAGATTTGCTAACCGTAGTGAAGCAACGAGATGATGAAAAG
TTGCTGGAGCGAGTGTTTGTTCTGGTTCGTTATGGTTGTATATATACCGGC

ACATTACGTGTAAATAACATTCCAGCTGGTCCATGGAAACATTTTTCTAT
GTTCAAACAGTTAACATCGCAACAAGTGATATGAAGCCGCTGCTGGCAGC
GCAAATTATTGGCGGCTTACCTGTTTCCCAAAATGCTAAAGCGGACCTGG
ATATAGCCTGTGGACCACTCACTTGGGAAGATGGTGAGTTTGATATTGAG
TTGATATAGAGTGTACTGCAGGGCTGATTCATATTGGCTAATAGCCATGG
AATAATGTGAGTTGTATTGATAAGTAATACCTTAACTCTGTACGGTACGAT
TTTTGGGACCGTCGTTGCGAGGAGCGTANCGACGAAGCAATCCAGTGGCC
TGAATTCGTTGAAGTAGCTCACTGATTCATTGATAACCCATATTTTTCCG
CTAAGAAACGACATGGAGTCGTGAATGAGTATCACTGAGCTGGCAGATAT
TTTAAATGGTTATTTTTCATGGAACAAGTCGCGAATCGAGTGTTTTGCGAC
GATGTTAATCTCTTTAATTCAAGTAAGAAAGGTCAATTTAACTGAAATTGC
ATGTGGGTTTTCTAGCCCTGCTAAAAAGATTCAAGGTACACAAGAATCA
AACCATTTTGAAAAACCACGCATGACTCAACCGGATCGCATTGAAAAACT
ACTGGTGCTATTGAACATTGCTTTTGTTGGGCCCCTAAAAATGGAGAGT
GGCGGCATGTTCAGAAGACAATTAAAATCAAAAAACATGGTCGCAAAGGG
GTAAGTTTTTTCGTTATGGACTTGATTTATTACATGATGCTGCGCTTAAT
GGCTGTCAGTTTATTCACCATTCTTTCTCCGATATACTTGGGTTTTTGTGC
ATTAGTCGCGTGATATGCGCTGCTGAATGAATTGGTCCAATAACAAGGAA
GAAGCATGCAAGTGAAACAACCAGCCATTTACATCATGGCGAATAAACGC
AATGGAACGATCTACACGGGTGTCACAAGCGATTTGATCAAACGTGTCTA
TGAACATAAATATGGGGATGTACCCGGGTTTACTCAAGAGCATGGATGTA
AGTTTCTTGTTACTATGAGCTCATGAAGACAGAACGAATCAAGCTGT

SEQ ID NO 5: 30A9

AGCTGATTCGTTTTTAGTTGTTTTTCTCGAGCTATGGCACTTATCATGTCT
TCAATGAGCTCATAGTAAACAAGAAACTTACATCCATGCTCTTGAGTAAAC
CCGGGTACATCCCCATATTTATGTTCAATAGACACGTTTGATCAAATCGCT
TGTGACACCCGTGTAGATTGTTCCATTGCGTTTATTCGCCATGATGTAAAT

GGCTGGTTGTTTCACTTGCATGCTTCTTCCTTGTTATTGGACCAATTCATT
CAGCAGCGCATATCACGCGACTAATGCACAAAAACCCAAGTATATCGGAG
AAAGAATGGTGAATAAACTGACAGCCATTAAGCGCAGCATCACGTAATAA
ATCAAGTCCATAACGAAAAAAACTTACCCCTTTGCGACCATGTTTTTGAT
TTTAATTGCCTTCTGAACATGCCGCCACTCTCCAGTTTTATGGGCCCAACA
AAAAGCAATGGTCAATAGCACCAGTAATTTTTCAATGCGATCGGTTGAGT
CATGTGTGTGTCTTCAAAATCGTTTGATTCTTGTGTACCTTGAATCTTGTT
TAGCAGGGCTAGAAAAACCACATGCAATCTCAGTTAAATTGACCGTTCTTA
CTTTGATTAAAGAGATTAACATCGTCGCAAAACACTCGATTCGCGACTTGT
TCCATGAAAAATAACCATTTAAAATATCTGCCAGATCAGTGATACTCATTC
ACGACTCCATGTCGTTTCTTAGCGGAAAAAATATGGATTATCAATGAATCA
GTGAGCTACTTCAACGAATTCAGGCCACTGGAATGGCTTCGTCGCTACGC
TCCTCGCAACGACGGTCCCAAAAATCGTACCGTACAGAGCTACAAAACAG
ACAAGGATAATCAGGAAGCCTTTTTATTTTGTCGCTTTGCTACCTCAAGAA
ATGTATACTGGCATTCTGTAACATTACCAACCATGTCATTTGTCATGACGG
GGTGTCGCTAAAACTTGAGACATGTAAAGTATTAAAATACTATAAGGCGA
AGAAGCAGATTAAATCGAAAGGGGAATTTCAGTAAACCGAATGCCACCGA
AAAGGATCCTAAATAAGGAAATAATAGAGATGGATCCCGTGTACATGATA
AACCACACCAGAGGCGAGCCTTTCTCGAAGAAAGACGAT

SEQ ID NO 6: 9F12

AATCAGCTGATTCGTTATGGGTTTATAGTCCATCTTGAATCATGTACAAAC
GAGAAATTGACCTTGAAAATAAGGCAATAAAAGTCCTGACTGAGGAAGAT
AACAAAAGCTATGAAAAACAGGTCAAAGCGCAATTAGATAATATTAAAAA
TGTTTTAGAATCACTTGAAATGAAAATAATTAGCCATTCTGAACAATGGGA
GAAAATTAGAAATCCCGCAATTCAAAAAGCGAAAGAATACTATTTAGGTG
ACATGGAAAAAGGTAACTGGGTAACTCAATGGGTAACGCAGTCCTGGATT
AATGAGCTATCCTCTCTTCATTCCACCAGTGAATTTATTTATGAGTTAAAT
GCTTTACCCCCAAGAAAAAGAGTACGTTTATTGAAGGTGAAGAACAAAG

TGTTGATACCACCTTGGAAAATTTAAGAAATCAAATTATTGATCGCTGTGG
TTTGCGAAGCATGATTGATGCGGAAGACAACTCTCCTGAACAACAATTATT
GAAAAAATCAATTGAATACTATGCCCACTCGCTGGCCATTTCAATGCAGC
GCTCCTTTTTAGTTAAGATTCCTGGAACTAATAATTATCAGGAATTTGACG
GCATCAATGACAAAGGGGAGATAATAGAACGCAAAGAAAACAATGGAACA
GGCAAAGATTATTTGCAAAGTAATTTTACCCAAAGAAAAATTAACGAGAAA
ATGAAGTTTTTCAGGGCAGTCAAAGCGTATCACCTCCATTTGATGTCCGAA
TTACCTGAAAATCCAGAACAATATAAAAACAGCTATATATTTATCAAAAAT
AACGATACTAAGGAGCTGTATTATATAAAACCTGATGGGAAATATGAAAA
AGTAAAAATTATCAAATTCGATTTAATTGAAGAAAAAATAGATAACAATAA
AGATGCGAATAAATTAATCCTTCTCAGTAAGGAAGAAATTAAAGAAATTGT
AACATTAAACGGAGGCCACTCCCCAGCAGAAGAGCACCATACTAAATTCA
CTGTTAACGATGTGGCTTTAAAACTAATGGGAGGCAAACCTGCCTATCCG
TTTGCTCGAAAATGGTTTGGTCCTAAATTTGATGAAGCCTTGAAACATCGA
ATGAACGTTGCTGCCAAATCCCTGCTCGTTGATGCAATAAATCATCCCAAA
ATTCACTTTGTATTTAACCGTTCACATGGAACGAATTGAAGATTCTCTGCG
AAGAAATTATTATAATTCTGACTTAATTAAAAAGGTCGAGTTTAAACGAGT
CTACGCGAGATGCGGGCGAATGAATACGCGCTTGTAAGAAGAACGAATTT
GAAGCCCACACTTTTTTCCATAATCGATGCGGAAAAACAACTTACGGAAC
GTGGCGTTACTATTCCAGAGTTTCCGATGAAGACTTTACGACGGTTC

SEQ ID NO 7: 35C6

CGAATCAGAGATTCGTCATGGGCATGCCCACCAGATGGACTTTATGAAAG
TTATGATTTCAAAATTTATTTACCGACCCCCAACGAAAAGCCTTCCTTAT
TCAAATCGACAATGAACTGGCCGGTTTTGCTTTATTAAATAAATTCGGAAT
CCAAGCCGTTGATTGGAGTATAGGGGAGTTTTTCATCCTGGCTAAATTCC
AAGGGAAAGGCATTGGTCAATCAGTAGCTCATCAACTATGGCGCACTCAT
CCAGGTGAATGGGAAATATCGGTCATTCCGGAAAACATACCGGCTTTATC
GTTTTGGCGCCAGGCTGTAATGACCTATACTCTTGAACGGTACAAAGAAC

AGATAAAGACAGTGAATTATGATATCCACCAAACCCAACGCATTATTTTAA
GTTTTAATACACAAAAACAGAACAAATCTTGTCATTCCATTCCAAAAAAAC
CTATTACAATAGATTTCGTGGATAATATCCCCAAATCTCTGGAAAAAAAGA
TGACAGAAGATCTGATAGCCTATGAAAGAACTCATGGTATTGATGTGAAC
TACAAGCGTTTTTCGATAGTACTATCTGAAGGAGGGGTGACTTGTGGCGT
TATTAATGCCTTTACTGCTTTTGCGGAAATATACATCGATGACATATGGGT
AGATAAAGCATATCGTGGTAAAGGATATGGCAGAAAATTACTGCAAACTC
TTGAGAATCATTTTAAAGGGCAAGGATTTAATAATATCAATTTATGTACCA
GCGCCTTTCAAGCACCGGAATTTTACAGAAAATGCGGATATACGGCTGAA
TTTACACGAATCAATCAAGCCAATCCCAAGCTTTCTAAAACCTTCTTTGTG
AAATTTTTTGATGATGAAAATCAAACACAAGGACTATTTTGAAAAACAGAA
CTTCTAAATAACCCCCACATCTTATTGATATTCAGAAGTTCTGAAAAAGTA
ATTTTCAATTAAAGAGACTAATTAAAGTGCCTATAAACCGTCTTATTCCAA
CTGATTACATTAGTTTGTTTTAAGAGAAAAATCCTGGGAAGTCAGAAACCA
ACGTCGAATATTTTTTTCGGAGTATTTATGATACTGGCACCAATCTCTAAT
AATAGAGGAATCTATATCTTCCGGGATGTCATGTATGCCTCGAGAAAAATT
TCCCTGTAAAAGCAATAAACCTCCCAGATTATGCAAATAAAACTCAGGGTC
TATAAAAATCATTGAAGTAGGATCATATAAGCAAAAATTAATAGGTTTTTT
ATCGTATAATACATCCTGAGCGTTGAAATTGGGGCCAAACTCATCATGAA
GCCCTAAATTCCCCAGGATTTTGCCATCAAACCAGGCTCTGGGATATTTGC
TCATGATATTTTTACCACCAGTTGCAGTTAAAATAATATCTGCTTTAGCCA
CTGCTTTTTCAAGCTTATCGCGATCCTCGGGAGAATACGAATCAGCTGATT

SEQ ID NO 8: 7C10

AATCTTTATTAGAGAACTCTTCTGGGTCTTAAGGCATGAATCATGGGGTTG
TGGAGAGAACAATCCATTGAGGCATTTTAGGCTTATGGTAAAAATGCAAA
TGCCTTAATGTATTCGGGTCAATTATATTATTGTTGATTTGCAAAACCCAG
ATAACACAAGATTGGGTAGAGTTCGCTGACAAAGCCACAAAATTTGCCTG
GCAGTTTGGTATTGGAAGCCTCTATGCCCTCAGTGACTCGTTGTTAATCGA

TATCAATTATAATTTTATTTCAATTGGCGAAGTGAGGAATTCAGGTCAGTA
TAATGCTATTGCAGCCAATAATACTCCAGCTTCAGGTGCGCCAACAAAATT
CACCAACGTGTATAGCAACCAGGCTGAAATTGGTATTCATTACCAATTTAA
TACTTAGCGCCAATTCAAGGCAGGTTGAAGTAAAAAGATGACATCAATGG
GAGCCACTGTTTAACCTGCCCATTGCTATCTACTCGGCTGAAATGAAAACT
ACCTTCGTACTCAGGTCTTTCCTGGTTTTTAGCTGCCTGGTACTTCACTCA
TAGTTTTCCTGGCAAATTTTCCCGTAATTTTACGGACTTTACCTCATGATG
GAAAAAGTGGGCAGTCAATCTTCCATGATGTTAAACGAGCGGTGCTTTAA
GTAGATTGTAATGTATATAAATCGTTGTCCTTGATTGCGTTTCGCTACATC
ATAGCTATAAGGTTAATAAATATTCCAAATAAATTGGGAAGTTGGCTTAAA
TGTGTCTATATTTGTTAGTATGTGAACACTTCCAGAATATCCAAACTCTAC
AGTAGGAATAAATTGAATTGATTTTAGAGTAAGAAAATGGATTGATAAACA
GATTTGAATGTTTCTTGTTAGCATCTCAATCAAGACAAACAAGGAAAGCAT
AATGTCTAAGACTATATATTTTTTCAGTAACAATTTGAAACTGGAAGGAAA
ACTGGAAGAACCAACAGGTCAATGCCTTGGTTATGTTTTATTTGCTCATTG
TTTTACCTGCGGAAAAGATATAGCTGCTGCAAGCAGAATTGCCAGCGCTT
TGGTTGCTAATGGATTTGCGGTGTTACGGTTTGATTTTACTGGCTTAGGTA
GCAGCGAAGGTTCCTTTTCAGAAACGAATTTTTCTTCAAATGTTGAAGATT
TAGTTGCTGCCGCTGACTATTTAAGGACTCATTATCAAGCACCTGTTCTTT
TGATAGGCCATAGTCTGACGATTCTCTGATTCGT

SEQ ID NO 9: 12A12

AGGGTCAGATCTTGCTCTTCGCCTTTATCCATAAAAGACGGCAAGCTTGG
AATTAACGCTTGATAATTATTTCCCCAACGCATTCCATTATCTTTCAAAAG
GAATTAACTAAGTCCAGGAGCAGCGATTGGTAAGACTTTTCGCGCATCGA
GTTTTCTGGTGGGCTCTATCACATCAACAGTCGAGGGAATAGAAAAGAAG
CGATCTATTTATCAGATGCAGACAGAGAGAATTTTTTATCTGTTTTGGGTG
ATGTTTGTTTAAAATATCAGTGGTTATGCCATTCGTATTGTCTGATGACAA
ATCATTACCATTTACTTATTGAAACTCCGCTCGGTAACCTTTCAAAAGGAA

TGCGCTATCTGAACGGGGTATACACTCAGAAATTTAATAAATCACACAAG
CGTATTGGGCACGTCCTGCAAGGTTGCTATAAATCTATATTGGTCACTACT
CAAGAATTTGCAGAATTATAAGACAACATTATATGCAGGAGGCAAAGGAC
AAGATCTGACCTACGCTCTAAACTATAATAATAGTTATAGATAATTGACTA
TTGGGATATTTTACATGAATTACCTATTGCTGATTATCCTGGGATTTGTA
ACTGCTCTTGGTTTTGCTGCGAATACACCTTGTTCTGGAAAAAGGGAGG
TATTTCTCATTGTTTAGGTGAATATTTCGTATGTAAAGATGGCTCANTAAG
CCAATCAAAAAACAAATGCATTGATCTAGATAAAAAAAATGGTCAATAAAT
TTCACTCAATTTTGCTTTTATTTCCCTTCTTTTAGTTGCACTGCCTATGC
AAATACCATTAGCGGATTGGTTATAAAAATATCAGATGGTGATACATTAAC
TATCCTCACTAAGGATAACGTTCAACTTAAAATTCGTTTATCAGAAATTGA
TGCACCAGAGAAAAAACAACCATTTGGAAATAAATCCAAGCAGTCGCTCT
CAAATCTTTGTTTTAATAAAGTAGCGATAGTTGATATTTTAAAAATTGATA
GATATGGCCGCTCTGTTGGCAGAGTTAAATGTGATAATGTTGATGCTAAT
GAATACCAAGTAAAAAATGGGCTTGCATGGGTGTATGATAAATATGTTAC
AGATCATTCGTTATATGCGCTTCAGGAACAAGCTAAAAGCAAGGGAATAG
GTTTGTGGTCGGAAAAATCCCCCATCCCTCCTGGATTTCAGACAAATTAGC
C

SEQ ID NO 10: 19H4

AAAGGAGACTTGGCAGCTCTCAAGAACTTGTTTGGTTGCAAGCAAAAACT
TCAACCCGGCTCTCTAATTAGCCTTGATAATACAACCTTTGCCTTTCCTTG
TAAGGGAGTTCCTACCCAATACTGGGATCTGGTAAATGGCTGGGAATTAC
AAAACAAGGTATCTATAGGTGAACCCATTAAATGGTCGGATATTAAGCAA
AAAAATGATTGTTAAAAACCCTACTTGGAACTCTCTTACCCCTCCACTTCC
TAAATTTGTTTCTTGTGATACAGGCGAGACAAATTTCCTGAAAGAAAAATT
AGGAGAAGATGGGAGGGTTTTATTGCTCAATTTGATCTAAAATCCACCTC
TGCTTTTTTATTTCAGCGTAAAAGTGATCAAAAAAAATTTTTATCAAGAA
AGTATCCGCTGAACATAAAAGACAGTATCAGCAGAGCGAACATTTGGCTC

AATTTATCGCTTGCCCAGATTATATAGTTAATATTGCAATTAACTGCGTTT
CTAATGAAGAAGAAAACTCACTCTATTATATTTATCCCTATATTCATGGAA
AACGCCTCTTTGCTGAACCAGAGGAAATAATAAGCCTTGCAACTGCATTA
GCAAAATTACATCTTAAAAAAAAATCTTATCCTGATCAACAATTGATTATT
AAAAATACTACGGAACGAACATCACAGCTTAATTTTATAAGAAAGGCTTTA
GCTAATGGATGTTATTCCTACATTCCATATTTCTCTTTTGTTAAAAAGATG
GCTCAACAATATGATTTTGATTGGATTAATCAAGAAGATGCCCAACCAATT
CATGGTGATTTAAATGCAGGAAATCTGTTACTATCCGAAAATAATATGATT
TGCTTTTTTGACTTTGAAGACGCATTACATAGTTTCCATCCAGTAGTATTA
GATTTGCTATTCGTCATTGAGCGTATTATTTTTAATCAAAATAGTTCGACT
GAACGGCTATTAAACCTTGGTCTCATGTTTATTCATGCATATAAAAAGGCT
GGTGGTACATACCGGTATAAAACGAGAGATGAATTTGGATTAACAATTCT
TGCTTTAAAAGCATTTTGCTTGTTGACTTTGCTAGCAGAGAAAAATAAAAA
TATACTGAACTCGGAATGGGATAAATTTTTAAATTAACTCAGAAAGCAGA
AAATGAACGGGATTTAATAAAAACAATTTTACAAGGATAAGAAAGATAGT
GTGAGAGTTCTTGTTACTGCACGGGATGTTGGTGCTGCATTAAACATTATT
GAGATAGTAAAAATACTTAAACAATATACAGGTGTTACTGTATATATTTAT
GCTCAACCTCCCGCTTCAAAATATTTCTTCGGGCTGGATTCAATCTGTA
TTTCAAGTTCCACTACCACCAACCCGATCCTCAAATGAATCCAATCATCTA
CTATCCTACGCCAATCAATTAATTGATAAATTAAATCCCGATATCATATTA
TCTGGATTATCATCTCCTGGCGAAGCTGGCATAGATGAAGCAATAATAGC
TGTGTGTCCCGCGCATATCAAAACATTCATCCTTCAAGATTTTGGGGAGA
AGTTAATTTTTTCTTTGAAAAATTAGCGGATTGCTATTTATGCATAGATCA
TCACGCTGCAGAAATTACAAAAAAACGATTTAATGCCAAAACCAGGGTGA
TAGGCTCCCCTCGCCATACCATTTTCAAACATAAAA

## Claims

1. A method for detecting a subgroup of *Legionella,* wherein said method involves a hybridization assay with a specific genetic marker selected from the group of markers as depicted in SEQ ID NO:1 - SEQ ID NO:9 or homologues thereof.

2. A method according to claim 1, wherein said subgroup of *Legionella* is serogroup 2-14 and the marker is 11B3 (SEQ ID NO: 1)

3. A method according to claim 1, wherein said subgroup of *Legionella* is serogroup 1A and the marker is 5G5 (SEQ ID NO: 2)

4. A method according to claim 1, wherein said subgroup of *Legionella* is serogroup 1B and the marker is selected from the group of 9G2, 8E11 and 30A9 (SEQ ID NO:3 - SEQ ID NO:5).

5. A method for detecting *Legionella,* wherein said method involves an hybridization assay with one or more incompatible marker pairs.

6. A method according to claim 5, wherein said marker pair is the pair 19H4 - 11B3.

7. A method for detecting a subgroup within serogroup 1 of *Legionella,* wherein said method involves an hybridization assay with one or more incompatible marker pairs.

8. A method according to claim 7, wherein said marker pair is selected from the group consisting of the pairs 5G5 - 9G2, 5G5 - 8E11, 5G5 - 30A9, 9F12 - 35C6, and 7C10 - 12A12.

9. A method according to any of the previous claims, wherein the markers are hybridized with genomic DNA of a sample, preferably fragmented genomic DNA.

10. A method according to any of the preceding claims, wherein the size of the specific genetic markers is from about 50 to about 2000 nucleotides, more preferably from about 500 to about 1800 nucleotides, most preferably from about 1000 to about 1500 nucleotides.

11. A method according to any of the preceding claims, wherein the method is used for determining the presence of a plurality of *Legionella* strains, the method involving a hybridization assay with at least two specific genetic markers selected from the group of markers as depicted in SEQ ID NO:1 - SEQ ID NO:9 or homologues thereof, wherein said markers are specific for a different strain.

12. Use of an incompatible marker pair for characterization of a sample suspected of harbouring multiple *Legionella* species.

13. A hybridization assay kit which comprises less than 50 nucleotides and which comprises at least one sequence selected from the group consisting of SEQ ID NO: 1 - SEQ ID NO:9 or homologues thereof.
